# EUROPÄISCHE PATENTSCHRIFT

(11) **EP 3 250 297 B1**
(45) Veröffentlichungstag und Bekanntmachung des Hinweises auf die Patenterteilung: **06.07.2022**
(21) Anmeldenummer: 15808354.3
(22) Anmeldetag: 10.12.2015
(51) Int. Cl.: A61B 5/0205, A61B 5/11, A61B 5/22, A61H 1/02, A63B 71/00, A63B 23/035, A63B 23/04, A63B 71/06, B25J 13/08, A63B 21/00, A63B 21/005, A61B 5/00, A63B 24/00

(54) **ROBOTERGESTÜTZTES TRAININGSSYSTEM**
ROBOT BASED TRAINING SYSTEM
SYTEME D'ENTRAINEMENT BASÉ SUR UN ROBOT

(30) Priorität: 26.01.2015 DE 102015000919
(43) Veröffentlichungstag der Anmeldung: 06.12.2017
(73) Patentinhaber: KUKA Deutschland GmbH, 86165 Augsburg (DE)
(72) Erfinder: KEIBEL, Andreas, 86161 Augsburg (DE); ARENBECK, Henry, 47198 Duisburg (DE); KOLDITZ, Melanie, 52066 Aachen (DE); ALBRACHT, Kirsten, 50858 Köln (DE); ABEL, Dirk, 52072 Aachen (DE); BRÜGGEMANN, Gert-Peter, 50858 Köln (DE)
(74) Vertreter: Schlotter, Alexander Carolus Paul
(86) Internationale Anmeldenummer: PCT/EP2015/002492
(87) Internationale Veröffentlichungsnummer: WO 2016/119805

(56) Entgegenhaltungen:
- WO-A1-2011/076240
- US-A1- 2008 161 733

## Beschreibung

Die vorliegende Erfindung betrifft ein Trainingssystem mit einer robotergeführten Betätigungsfläche, ein Verfahren zum Regeln eines Roboters des Trainingssystems sowie ein Computerprogrammprodukt zur Durchführung des Verfahrens.

Aus der WO 2011/076240 A1 ist eine Physiotherapievorrichtung mit einem Roboter bekannt, der eine Betätigungsfläche führt. Die Betätigungsfläche kann längs einer vorgegebenen Trajektorie geführt werden, um einen Nutzer passiv zu trainieren. Eine sechsdimensionale Kraft-Momenten-Messung ermöglicht zudem ein isometrisches, exzentrisches oder konzentrisches Training, indem der Roboter auf die Betätigungsfläche eine Kraft ausübt, die einer vom Nutzer aufgeprägten Kraft entspricht (isometrisches Training), diese geringfügig übersteigt, so dass sich die Betätigungsfläche gegen den Widerstand des Nutzers bewegt (exzentrisches Training), oder diese geringfügig unterschreitet, so dass der Nutzer die Betätigungsfläche gegen den Widerstand des Roboters bewegt (konzentrisches Training).

Als Sicherheitssystem ist ein Notaus- oder Totmann-Schalter vorgesehen, der den Roboter unverzüglich stoppt.

Aus der US 2008/161733 A1 ist ein Rehabilitationsvorrichtung mit wenigstens drei Freiheitsgraden bekannt, die mehrere Bremsen, damit korrelierte Oberflächen und einen Motor aufweist, der die Bremsen selektiv auf die Oberflächen zustellt, um einen variablen Widerstand in den Freiheitsgraden zu bewirken.

Eine Aufgabe der vorliegenden Erfindung ist es, ein robotergestütztes Training zu verbessern.

Diese Aufgabe wird durch ein Trainingssystem mit den Merkmalen des Anspruchs 1 gelöst. Ansprüche 11, 12 stellen ein Verfahren zum Regeln des Roboters eines hier beschriebenen Trainingssystems bzw. ein Computerprogrammprodukt, insbesondere einen Datenträger bzw. ein Speichermedium, zur Durchführung des Verfahrens unter Schutz. Die Unteransprüche betreffen vorteilhafte Weiterbildungen.

Nach einem Aspekt der vorliegenden Erfindung weist ein Trainingssystem einen Roboter auf. Der Roboter weist in einer Ausführung einen oder mehrere Arme mit jeweils wenigstens sechs, insbesondere elektromotorisch, aktuierten Gelenken, insbesondere Drehgelenken, insbesondere mit paarweise aufeinander senkrecht stehenden oder parallelen Drehachsen, auf. In einer Weiterbildung weist der Roboter wenigstens einen Arm mit wenigstens sieben Gelenken auf, wobei diese Redundanz vorteilhaft insbesondere zur Vermeidung singulärer Posen genutzt werden kann.

In einer Ausführung weist das Trainingssystem wenigstens eine Betätigungsfläche auf, die, insbesondere lösbar, an dem Roboter, insbesondere einem Roboterflansch, der gegenüber einer, insbesondere umgebungsfesten, Roboterbasis die durch alle Gelenke des Roboters definierten Freiheitsgrade aufweist, befestigbar ist, insbesondere befestigt und somit durch den Roboter geführt ist bzw. wird.

Die Betätigungsfläche ist als Nutzer-Schnitt- bzw. Kontaktstelle mit dem Roboter vorgesehen bzw. eingerichtet. Sie kann in einer Ausführung, wenigstens im Wesentlichen, eben sein, beispielsweise eine Plattform zum Abstützen eines oder beider Füße aufweisen. Gleichermaßen kann eine Betätigungsfläche auch, insbesondere zylinderförmig, gekrümmt sein, beispielsweise einen Griff zum Halten mit einer oder beiden Händen aufweisen. In einer Ausführung entspricht die Betätigungsfläche einer Kontaktfläche eines Sportgerätes, dessen Einsatz der Nutzer mit dem Trainingssystem trainieren will, beispielsweise einem Schaft eines Leichtathletikspeeres, einem Griff eines Golfschlägers oder dergleichen. In einer Ausführung weist die Betätigungsfläche einen Überzug aus Kunststoff oder Gummi und/oder eine Oberflächenstrukturierung auf. Hierdurch kann vorteilhafterweise eine Griffigkeit bzw. ein Kontakt des Nutzers verbessert werden. In einer Ausführung ist die Betätigungsfläche zum Kontakt mit einem oder zwei Füßen, Händen und/oder sonstigen Körperteilen, beispielsweise Rücken, Schulter oder dergleichen, vorgesehen bzw. eingerichtet bzw. wird im Betrieb durch diese kontaktiert.

In einer Ausführung weist das Trainingssystem ein Krafterfassungsmittel zum Ermitteln einer Beaufschlagung der Betätigungsfläche auf. Die Beaufschlagung umfasst in einer Ausführung eine Kraft in einer oder mehreren, insbesondere drei, vorzugsweise zueinander orthogonalen, Richtungen und/oder ein Drehmoment in einer oder mehreren, insbesondere drei, vorzugsweise zueinander orthogonalen, Richtungen. Zur kompakteren Darstellung wird vorliegend auch ein antiparalleles Kräftepaar bzw. Drehmoment verallgemeinernd als (eine) Kraft bezeichnet.

In einer Ausführung weist das Krafterfassungsmittel einen, insbesondere mehr-, vorzugsweise sechsdimensionalen, Kraft- und/oder Momentensensor auf, der in einer Ausführung zwischen dem Roboterflansch und der Betätigungsfläche, insbesondere einer Kupplung zur lösbaren Befestigung der Betätigungsfläche an dem Roboterflansch, angeordnet sein kann.

Zusätzlich oder alternativ weist das Krafterfassungsmittel einen oder mehrere Kraft-, insbesondere Drehmomentsensoren, in einem oder mehreren, insbesondere allen, Gelenken des Roboters auf. Insbesondere unter Berücksichtigung eines mechanischen Modells des Roboters, insbesondere seiner Trägheiten, kann hieraus ebenfalls eine ein- oder mehrdimensionale Beaufschlagung der robotergeführten Betätigungsfläche ermittelt werden.

Nach einem Aspekt der vorliegenden Erfindung weist das Trainingssystem ein Aktivitätserfassungsmittel zum Ermitteln einer biomechanischen und/oder kardiovaskulären Belastung des Nutzers, insbesondere auf Basis einer von dem Krafterfassungsmittel ermittelten Beaufschlagung der Betätigungsfläche, auf.

Eine biomechanische Belastung umfasst, insbesondere ist, in einer Ausführung eine, insbesondere mechanische, Belastung bzw. Beanspruchung des Stütz- und/oder Bewegungsapparats, insbesondere von Gelenken, Muskeln, Bändern und/oder Sehnen des Nutzers, insbesondere Gelenken seines Bewegungsapparates, insbesondere Skeletts. Eine mechanische Belastung des Stütz- und Bewegungsapparates umfasst in einer Ausführung Kräfte, Drehmomente, Spannungen und/oder Dehnungen an den biologischen Strukturen des Stütz- und Bewegungsapparates, insbesondere an Muskeln, Bändern, Sehnen, Knorpel, Knochen und/oder Gelenkflächen, insbesondere die biomechanischen Parameter Gelenkmomente und/oder Gelenkkräfte. Entsprechend umfasst in einer Ausführung eine biomechanische Belastung beispielsweise eine Belastung, insbesondere der Gelenkflächen, eines Hüft-, Knie- und/oder Fußgelenks, des Hüftstreckers und/oderbeugers, der Oberschenkel- und/oder Wadenmuskeln, der Außen-, Innen- und/oder Kreuzbänder von Knie- und/oder Fußgelenken, der Achillessehne oder dergleichen. Eine kardiovaskuläre Belastung umfasst, insbesondere ist, in einer Ausführung eine Belastung bzw. Beanspruchung des kardiovaskulären bzw. Herz-Kreislauf-Systems des Nutzers.

In einer Ausführung umfasst eine biomechanische und/oder kardiovaskulären Belastung insbesondere eine akute bzw. während der Betätigung des Trainingssystems auftretende Belastung. Zusätzlich oder alternativ kann eine biomechanische und/oder kardiovaskulären Belastung insbesondere eine Langzeit- bzw. nach der Betätigung des Trainingssystems auftretende Belastung umfassen.

Eine biomechanische Belastung kann insbesondere mechanische Kräfte und/oder Momente und/oder eine (potentielle) Schädigung bzw. einen Verschleiß, insbesondere eines Bewegungsapparates und/oder von Gewebestrukturen des Nutzers, umfassen. Zusätzlich oder alternativ kann eine biomechanische Belastung im Sinne der vorliegenden Erfindung auch eine Trainingswirkung, insbesondere eine Verbesserung einer Leistungsfähigkeit des Nutzers gegenüber einem Ausgangszustand, umfassen. Auch eine solche Trainingswirkung, die biologisch eine Reaktion auf eine mechanische Belastung darstellt, wird vorliegend verallgemeinernd als biomechanische Belastung bezeichnet.

Beaufschlagt der Nutzer die Betätigungsfläche, so resultiert hieraus als Reaktion eine biomechanische Belastung, insbesondere seines Bewegungsapparates, und/oder eine kardiovaskuläre Belastung, insbesondere seines Herz-Kreislauf-Systems. Entsprechend kann, insbesondere modellgestützt, auf Basis einer ermittelten Beaufschlagung der Betätigungsfläche auch eine biomechanische und/oder kardiovaskulären Belastung des Nutzers ermittelt werden.

Bisherige Trainingseinrichtungen, insbesondere auch die eingangs genannte WO 2011/076240 A1, berücksichtigen jedoch nicht die biomechanische und/oder kardiovaskulären Belastung des Nutzers, sondern konzentrieren sich auf die (absolute) Beaufschlagung der Betätigungsfläche selber, indem sie beispielsweise eine bestimmte Kraft auf die Betätigungsfläche aufprägen.

Dies kann jedoch den Nutzer, insbesondere seinen Bewegungsapparat und/oder sein Herz-Kreislauf-System, ungünstig belasten, insbesondere überlasten oder unter Trainingsgesichtspunkten suboptimal belasten. Wird beispielsweise in einer Funktionsstemme nur die konstante Kraft in einer Verfahrrichtung vorgegeben, so kann dies die Muskeln je nach wirkenden Hebelarmen unter- oder überfordern. Zusätzlich kann beispielsweise das Knie überlastet werden, wenn die Verfahrrichtung nicht mit der Bein- bzw. Kniegelenkachse korreliert.

Daher weist nach einem Aspekt der vorliegenden Erfindung das Trainingssystem ein Steuermittel auf, das den Roboter, insbesondere seine Antriebe, auf Basis einer vorgegebenen und der ermittelten biomechanischen und/oder kardiovaskulären Belastung des Nutzers regelt bzw. hierzu, insbesondere hard- und/oder softwaretechnisch eingerichtet ist.

Auf diese Weise kann vorteilhaft die Gefahr einer biomechanischen und/oder kardiovaskulären Fehl-, insbesondere Überlastung reduziert werden. So können etwa in einem Kniegelenk des Nutzers wirkende Kräfte und Momente auf Basis der ermittelten Beaufschlagung der Betätigungsfläche ermittelt und mit vorgegebenen, insbesondere gewünschten und/oder zulässigen, Belastungen verglichen werden. Dann kann das Steuermittel den Roboter derart regeln, dass die im Kniegelenk des Nutzers wirkenden Kräfte und Momente sich den gewünschten Belastungen näheren oder die zulässigen Belastungen nicht übersteigen.

Zusätzlich oder alternativ kann so ein Trainingsreiz verbessert werden. So kann exemplarisch auf Basis der ermittelten Beaufschlagung der Betätigungsfläche eine muskuläre Beanspruchung, etwa im Kniestrecker, ermittelt und mit einem vorgegebenen optimalen Trainingsreiz verglichen werden. Dann kann das Steuermittel den Roboter derart regeln, dass die im Kniestrecker des Nutzers wirkenden Kräfte sich der gewünschten Trainingsbelastung nähern, beispielsweise bei größer werdendem Hebelarm eine (Gegen)Kraft des Roboters auf die Betätigungsfläche erhöhen oder dergleichen.

Wie vorstehend ausgeführt, kann eine biomechanische Belastung im Sinne der vorliegenden Erfindung mechanische Kräfte und/oder Momente und/oder eine Trainingswirkung umfassen. Entsprechend kann in einer Ausführung das Steuermittel insbesondere vorgegebene und ermittelte Kräfte und/oder Momente in Gelenken, Muskeln, Bändern und/oder Sehnen des Nutzers und/oder eine vorgegebene und eine ermittelte, insbesondere muskuläre, gewebliche und/oder motorische, Trainingswirkung vergleichen und den Roboter auf Basis dieser vorgegebenen und ermittelten biomechanischen Belastung regeln bzw. hierzu, insbesondere hard-und/oder softwaretechnisch eingerichtet sein. Allgemein regelt somit das Steuermittel den Roboter in einer Ausführung derart, dass eine Differenz zwischen der vorgegebenen und der ermittelten biomechanischen und/oder kardiovaskulären Belastung des Nutzers sich reduziert, bzw. ist hierzu, insbesondere hard- und/oder softwaretechnisch eingerichtet.

In einer Ausführung ermittelt das Aktivitätserfassungsmittel die Belastung des Nutzers auf Basis wenigstens eines biomechanischen und/oder kardiovaskulären Modells bzw. ist hierzu, insbesondere hard- und/oder softwaretechnisch eingerichtet. Das biomechanische bzw. kardiovaskuläre Modell verknüpft in einer Ausführung eine Beaufschlagung der Betätigungsfläche mit einer biomechanischen bzw. kardiovaskulären Belastung des Nutzers, insbesondere in Form einer relationalen Verknüpfung, insbesondere einer ein- oder mehrdimensionalen Abbildung.

In einer Ausführung weist das Aktivitätserfassungsmittel mehrere biomechanische und/oder kardiovaskuläre Modelle, insbesondere Modellmodule, auf, die in einer Weiterbildung unterschiedliche Teile des Bewegungsapparates des Nutzers abbilden und/oder unterschiedliche Komplexitätsgrade aufweisen. Dann erstellt in einer Ausführung das Aktivitätserfassungsmittel wahlweise, insbesondere je nach Anwendungsfall, insbesondere Trainingsplan, aus diesen Modulen jeweils ein biomechanisches bzw. kardiovaskuläres Modell, auf dessen Basis es dann die Belastung des Nutzers ermittelt. In einer Ausführung sind ein oder mehrere biomechanische und/oder kardiovaskuläre Modelle objektorientiert implementiert, was insbesondere deren Zusammenbindung erleichtern kann.

In einer Ausführung sind ein oder mehrere biomechanische und/oder kardiovaskuläre Modelle parametrierbar, insbesondere, um sie individuell an einen Nutzer anzupassen. Die Parameter des Modells werden in einer Ausführung vom Nutzer oder einem Trainer eingegeben oder aus einer Datenbank ermittelt, insbesondere durch Identifikation einer Nutzeridentität und Abrufen von Parametern, die mit dieser Nutzeridentität verknüpft sind, aus einem Speichermittel.

Zusätzlich oder alternativ können ein oder mehrere der Parameter auch durch das Trainingssystem selber ermittelt, insbesondere identifiziert oder abgeschätzt, werden. So kann beispielsweise ein maximaler Bewegungsbereich eines oder mehrerer Gelenke und/oder eine maximale Kraft einer oder mehrerer Muskeln des Nutzers durch ein- oder mehrmaliges Bewegen der Betätigungsfläche, insbesondere gegen einen vorgegebenen Widerstand, ermittelt werden.

In einer Ausführung ermittelt das Aktivitätserfassungsmittel die biomechanische und/oder kardiovaskuläre Belastung des Nutzers zusätzlich oder alternativ auf Basis eines ermittelten Zustands des Nutzers bzw. ist hierzu, insbesondere hard- und/oder softwaretechnisch eingerichtet. Insbesondere kann in einer Ausführung das biomechanische und/oder kardiovaskuläre Modell eine Beaufschlagung der Betätigungsfläche und einen ermittelten Zustand des Nutzers mit einer biomechanischen bzw. kardiovaskuläre Belastung des Nutzers verknüpfen, insbesondere in Form einer relationalen Verknüpfung, insbesondere einer ein- oder mehrdimensionalen Abbildung.

Der Zustand des Nutzers kann insbesondere eine Position, Geschwindigkeit und/oder Beschleunigung einer oder mehrerer Referenzen des Nutzers, insbesondere von Gelenkpunkten oder -achsen, umfassen, insbesondere sein. In einer Ausführung wird der Zustand des Nutzers (auch) mittels Ultraschall ermittelt. Entsprechend weist in einer Ausführung das Aktivitätserfassungsmittel wenigstens einen Ultraschallsensor auf.

So können beispielsweise auf Basis erfasster Positionen von am Nutzer angeordneten Markern und/oder auf Basis von in einem Bild des Nutzers durch eine Bilderkennung identifizierten Positionen von Referenzen, insbesondere auf Basis des biomechanischen und/oder kardiovaskulären Modells, die Positionen von Gelenken und/oder Muskeln des Nutzers ermittelt und so dort wirkende Belastungen ermittelt werden.

Entsprechend ermittelt das Aktivitätserfassungsmittel in einer Ausführung den Zustand des Nutzers auf Basis einer erfassten, insbesondere mehrdimensionalen, Position und/oder Beschleunigung des Nutzers, insbesondere einer oder mehrerer Referenzen des Nutzers, bzw. ist hierzu, insbesondere hard- und/oder softwaretechnisch eingerichtet. Insbesondere hierzu kann es einen oder mehrere am Nutzer angeordnete, insbesondere inertiale, Positionssensoren und/oder Beschleunigungssensoren aufweisen. Die Sensoren können aktiv oder passiv sein bzw. aktiv Daten erfassen und übermitteln oder passiv durch entsprechende Erfassungsmittel erfasst werden.

Zusätzlich oder alternativ kann das Aktivitätserfassungsmittel einen oder mehrere, insbesondere roboter- oder umgebungsfeste, Raumüberwachungssensoren aufweisen, insbesondere Lichtschranken, Scanner, Kameras oder dergleichen. Auch hierdurch kann insbesondere eine, insbesondere mehrdimensionale, Position und/oder Beschleunigung des Nutzers, insbesondere einer oder mehrerer Referenzen des Nutzers, ermittelt werden, insbesondere durch eine Bilderkennung.

Der Zustand des Nutzers kann zusätzlich oder alternativ insbesondere Nerven-und/oder Muskelaktivitäten des Nutzers umfassen. Entsprechend ermittelt das Aktivitätserfassungsmittel in einer Ausführung den Zustand des Nutzers auf Basis einer erfassten, insbesondere mehrdimensionalen, Nerven- und/oder Muskelaktivität des Nutzers bzw. ist hierzu, insbesondere hard- und/oder softwaretechnisch eingerichtet. Insbesondere hierzu kann es einen oder mehrere am Nutzer angeordnete EMG-Sensoren aufweisen.

Durch die Berücksichtigung der Nerven- und/oder Muskelaktivitäten kann vorteilhafterweise die Präzision erhöht und/oder eine Redundanz eines biomechanischen Modells aufgelöst werden.

Der Zustand des Nutzers kann zusätzlich oder alternativ insbesondere kardiovaskuläre Aktivitäten des Nutzers umfassen. Entsprechend ermittelt das Aktivitätserfassungsmittel in einer Ausführung den Zustand des Nutzers auf Basis einer erfassten, insbesondere mehrdimensionalen, kardiovaskulären Aktivität des Nutzers bzw. ist hierzu, insbesondere hard- und/oder softwaretechnisch eingerichtet. Insbesondere hierzu kann es einen oder mehrere am Nutzer angeordnete Sensoren zum Ermitteln eines ein- oder mehrdimensionalen kardiovaskulären Parameters, insbesondere Blutdruckwertes, Pulswertes, Bluatsauerstoffwertes oder dergleichen aufweisen.

Durch die Berücksichtigung der kardiovaskuläre Aktivitäten kann vorteilhafterweise die Präzision und/oder Sicherheit beim Training erhöht werden.

Der Zustand des Nutzers kann zusätzlich oder alternativ insbesondere Abmessungen biologischer Strukturen, insbesondere von Muskeln, Sehnen, Bändern und dergleichen, des Nutzers umfassen. Entsprechend ermittelt das Aktivitätserfassungsmittel in einer Ausführung den Zustand des Nutzers auf Basis einer erfassten, insbesondere mehrdimensionalen, Abmessung einer biologischen Struktur des Nutzers bzw. ist hierzu, insbesondere hard- und/oder softwaretechnisch eingerichtet. Insbesondere hierzu kann es einen oder mehrere, insbesondere nichtinvasive, Sensoren zum Ermitteln einer ein- oder mehrdimensionalen Abmessung einer biologischen Struktur, insbesondere von Muskeln, Sehnen, Bändern und dergleichen, des Nutzers oder dergleichen aufweisen. In einer Weiterbildung weist der Sensor ein bildgebendes und/oder -verarbeitendes Mittel zum Erfassen der Abmessung der biologischen Struktur auf.

So kann beispielsweise das Aktivitätserfassungsmittel bzw. dessen Sensor in einer Ausführung eine Länge einer Patellarsehne oder Achillessehne mittels Ultrasonographie als Abmessung einer biologischen Struktur erfassen bzw. ermitteln, hieraus eine Dehnung der Patellar- bzw. Achillessehne als Zustand des Nutzers ermitteln, und daraus eine, insbesondere biomechanische, Belastung des Nutzers ermitteln bzw. hierzu, insbesondere hard- und/oder softwaretechnisch eingerichtet sein.

In einer Ausführung regelt das Steuermittel, eine Kraft, insbesondere deren Richtung und/oder Größe bzw. Betrag, die der Roboter auf die robotergeführte Betätigungsfläche, insbesondere minimal, maximal oder aktuell, ausübt bzw. aufprägt, auf Basis der vorgegebenen und der ermittelten biomechanischen und/oder kardiovaskulären Belastung des Nutzers bzw. ist hierzu, insbesondere hard- und/oder softwaretechnisch eingerichtet. Wie vorstehend erläutert, wird auch ein Drehmoment vorliegend verallgemeinernd als Kraft bezeichnet.

Das Steuermittel kann insbesondere eine Größe und/oder Richtung einer Kraft, mit der der Roboter die Betätigungsfläche beaufschlagt, insbesondere bewegt, bzw. die er einer Bewegung der Betätigungsfläche entgegensetzt, derart regeln, dass eine ermittelte biomechanische und/oder kardiovaskulären Belastung des Nutzers sich einer vorgegebenen biomechanischen bzw. kardiovaskulären Belastung des Nutzers nähert bzw. gegen diese strebt.

Wird beispielsweise eine biomechanische Überlastung des Kniegelenkes ermittelt, kann das Steuermittel die Kraft, mit der der Roboter die Betätigungsfläche beaufschlagt, reduzieren und/oder ihre Richtung so ändern, dass die biomechanische Belastung des Kniegelenkes reduziert wird. Insbesondere kann das Steuermittel durch entsprechende Ausrichtung der vom Roboter ausgeübten Kraft eine biomechanische Belastung in eine günstige Achse verschieben.

Zusätzlich oder alternativ regelt das Steuermittel in einer Ausführung eine Bewegung der robotergeführten Betätigungsfläche durch den Roboter, insbesondere eine Bewegungsrichtung und/oder Geschwindigkeit der robotergeführten Betätigungsfläche, auf Basis der vorgegebenen und der ermittelten biomechanischen und/oder kardiovaskulären Belastung des Nutzers bzw. ist hierzu, insbesondere hard- und/oder softwaretechnisch eingerichtet.

Das Steuermittel kann insbesondere eine Geschwindigkeit und/oder Richtung einer Bewegung der Betätigungsfläche durch den Roboter derart regeln, dass eine ermittelte biomechanische und/oder kardiovaskulären Belastung des Nutzers sich einer vorgegebenen biomechanischen bzw. kardiovaskulären Belastung des Nutzers nähert bzw. gegen diese strebt.

Wird wiederum beispielsweise eine biomechanische Überlastung des Kniegelenkes ermittelt, kann das Steuermittel die Bewegungsrichtung der robotergeführten Betätigungsfläche so ändern, dass die biomechanische Belastung des Kniegelenkes reduziert wird.

In einer Ausführung regelt das Steuermittel den Roboter adaptiv. Insbesondere können Regelparameter und/oder -strukturen während und/oder nach einer Betätigung des Trainingssystems durch einen, insbesondere identifizierten, Nutzer, automatisiert verändert werden, insbesondere auf Basis der während der Betätigung ermittelten biomechanischen und/oder kardiovaskulären Belastung.

Nach der vorliegenden Erfindung weist das Trainingssystem ein Sicherheitsmittel zum, insbesondere redundanten, insbesondere diversitären, Überwachen der Beaufschlagung der Betätigungsfläche, der ermittelten biomechanischen und/oder kardiovaskulären Belastung des Nutzers und/oder eines Zustands des Roboters auf. In einer Weiterbildung erfasst das Sicherheitsmittel hierzu die Beaufschlagung der Betätigungsfläche und/oder den Zustand, insbesondere eine, insbesondere mehrdimensionale, Position, Geschwindigkeit und/oder Beschleunigung, des Nutzers und/oder des Roboters, zweikanalig bzw. ist hierzu, insbesondere hard- und/oder softwaretechnisch eingerichtet.

Durch die Überwachung der Beaufschlagung der Betätigungsfläche kann insbesondere eine (absolute, von seiner Biomechanik unabhängige) Überlastung des Nutzers erkannt bzw. vermieden werden. Durch die Überwachung des Zustands des Roboters kann insbesondere eine mögliche Kollision und/oder Fehlfunktion erkannt und hierauf reagiert werden. Durch die Überwachung der ermittelten biomechanischen und/oder kardiovaskulären Belastung des Nutzers kann vorteilhafterweise eine Überlastung insbesondere auch dann erkannt und entsprechend vermieden werden, wenn die Beaufschlagung der Betätigungsfläche an sich (noch) in einem zulässigen Bereich liegt. So kann beispielsweise auf Basis der Beaufschlagung der Betätigungsfläche und eines Zustandes, insbesondere einer Position, des Nutzers, auf Basis des biomechanischen Modells erfasst werden, dass ein Kniegelenk - etwa aufgrund einer Achsenfehlstellung - überlastet wird, obwohl die Absolutkraft auf die Betätigungsfläche noch in einem an sich zulässigen Bereich liegt.

Falls eine unzulässige Beaufschlagung der Betätigungsfläche oder eine unzulässige biomechanische und/oder kardiovaskuläre Belastung des Nutzers oder ein unzulässiger Zustand des Roboters ermittelt wird, löst das Sicherheitsmittel in einer Ausführung eine Fehlerreaktion aus bzw. ist hierzu, insbesondere hard- und/oder softwaretechnisch eingerichtet.

In einer Weiterbildung führt das Sicherheitsmittel eine Ausgleichsbewegung der robotergeführten Betätigungsfläche durch, falls eine unzulässige Beaufschlagung der Betätigungsfläche oder eine biomechanischen und/oder kardiovaskuläre Belastung des Nutzers oder ein unzulässiger Zustand des Roboters ermittelt wird bzw. ist hierzu, insbesondere hard- und/oder softwaretechnisch eingerichtet, insbesondere in eine vorgegebene Ausgangsstellung.

Durch eine solche Ausgleichsbewegung kann, insbesondere im Vergleich zu einem unverzüglichen Stoppen des Roboters in einer ergonomisch ungünstigen Pose bzw. Situation, eine Gefahr einer Überlastung oder eines Einklemmens des Nutzers reduziert werden. Wird beispielsweise ermittelt, dass die Beaufschlagung der Betätigungsfläche einen vorgegebenen Maximalwert übersteigt, so kann anstelle eines bloßen Stopps des Roboters die robotergeführte Betätigungsfläche in eine Ausgangsstellung verfahren werden, in der der Nutzer nicht überlastet wird und/oder das Trainingssystem besser verlassen kann.

Nach einem Aspekt der vorliegenden Erfindung weist das Trainingssystem zwei oder mehr, insbesondere unterschiedliche, Betätigungsflächen auf, die wahlweise mit dem Roboter koppelbar sind, insbesondere gekoppelt sind bzw. werden. Hierdurch können vorteilhaft auf den Nutzer und/oder das Training abgestimmte Betätigungsflächen zur Verfügung gestellt werden, beispielsweise Griffe mit unterschiedlichen Größen, verschiedene Plattformen oder dergleichen.

In einer Weiterbildung identifiziert das Steuermittel die jeweils robotergeführte bzw. an den Roboter(flansch) gekoppelte Betätigungsfläche und regelt den Roboters auf Basis der identifizierten robotergeführten Betätigungsfläche bzw. ist hierzu, insbesondere hard- und/oder softwaretechnisch eingerichtet.

In einer Weiterbildung weisen die Betätigungsflächen, insbesondere elektromagnetisch auslesbare, Identifikationsmarkierungen und das Steuermittel ein Mittel zum, insbesondere elektromagnetischen, Erfassen der Identifikationsmarkierungen auf. Die Identifikationsmarkierungen können insbesondere RFID-Transponder umfassen, insbesondere sein.

In einer Weiterbildung wechselt das Trainingssystem, insbesondere das den Roboter regelnde Steuermittel, vollständig oder teilweise automatisiert, die robotergeführte Betätigungsfläche, insbesondere gegen eine andere, wahlweise mit dem Roboter koppelbare Betätigungsfläche bzw. ist hierzu, insbesondere hard- und/oder softwaretechnisch eingerichtet.

In einer Ausführung identifiziert das Steuermittel den Nutzer, insbesondere berührungslos, insbesondere mittels RFID, und regelt den Roboters auf Basis des identifizierten Nutzers bzw. ist hierzu, insbesondere hard- und/oder softwaretechnisch eingerichtet.

Insbesondere können so nutzerindividuelle Trainingspläne und/oder Parameter des biomechanischen Modells zur Regelung des Roboters verwendet werden. Ein Regeln kann insbesondere auch ein Sperren einer Bewegung des Roboters umfassen. Entsprechend kann die Identifikation auch zur Autorisierung eines Trainings mit dem Trainingssystem verwendet werden.

In einer Ausführung weist das Trainingssystem ein ein- oder mehrteiliges Fixiermittel zum Fixieren des Nutzers an der robotergeführten Betätigungsfläche und/oder an einer, insbesondere verstellbaren, Nutzer-Positioneinrichtung, insbesondere einer Stand- und/oder Sitzfläche und/oder Lehne, auf. Hierdurch kann vorteilhaft ein Training verbessert werden.

In einer Ausführung weist das Trainingssystem ein Ausgabemittel zum, insbesondere optischen bzw. visuellen, haptischen und/oder akustischen, Ausgeben einer Rückmeldung auf Basis der ermittelten biomechanischen und/oder kardiovaskulären Belastung auf. Hierdurch kann dem Nutzer ein computergestütztes Feedback der biomechanischen bzw. kardiovaskulären Belastung, insbesondere einer Trainingswirkung, gegeben und diese so vorteilhaft verbessert werden.

Ein Mittel im Sinne der vorliegenden Erfindung kann hard- und/oder softwaretechnisch ausgebildet sein, insbesondere eine, vorzugsweise mit einem Speicher- und/oder Bussystem daten- bzw. signalverbundene, insbesondere digitale, Verarbeitungs-, insbesondere Mikroprozessoreinheit (CPU) und/oder ein oder mehrere Programme oder Programmmodule aufweisen. Die CPU kann dazu ausgebildet sein, Befehle, die als ein in einem Speichersystem abgelegtes Programm implementiert sind, abzuarbeiten, Eingangssignale von einem Datenbus zu erfassen und/oder Ausgangssignale an einen Datenbus abzugeben. Ein Speichersystem kann ein oder mehrere, insbesondere verschiedene, Speichermedien, insbesondere optische, magnetische, Festkörper- und/oder andere nicht-flüchtige Medien aufweisen. Das Programm kann derart beschaffen sein, dass es die hier beschriebenen Verfahren verkörpert bzw. auszuführen imstande ist, sodass die CPU die Schritte solcher Verfahren ausführen kann und damit insbesondere den Roboter regeln kann.

Ein Training im Sinne der vorliegenden Erfindung kann insbesondere eine Verbesserung von Gewebestrukturen, insbesondere Muskeln, Sehnen und/oder Bändern, des Nutzers umfassen bzw. anstreben. Zusätzlich oder alternativ kann es auch eine nervöse, insbesondere koordinative, Verbesserung des Nutzers umfassen bzw. anstreben. Entsprechend kann die vorgegebene biomechanische Belastung des Nutzers insbesondere auf Basis einer angestrebten Verbesserung von Gewebestrukturen und/oder auf Basis einer angestrebten nervöse, insbesondere koordinative, Verbesserung vorgegeben werden bzw. sein.

Eine Überlastung im Sinne der vorliegenden Erfindung kann insbesondere ein Überschreiten einer, insbesondere definierten bzw. vorgegebenen, Grenzbelastung umfassen, insbesondere sein.

In einer Ausführung wird der Roboter zusätzlich auch auf Basis eines vorgegebenen, insbesondere nutzerspezifischen bzw. -indivuellen, Bewegungsausmaßes geregelt. Entsprechend ist das Steuermittel in einer Ausführung zum Regeln des Roboters auf Basis eines vorgegebenen, insbesondere nutzerspezifischen bzw. -individuellen, Bewegungsausmaßes ausgebildet bzw., insbesondere hard- und/oder softwaretechnisch, eingerichtet. Auf diese Weise können vorteilhafte insbesondere Therapievorgaben bzw. Einschränkungen des Bewegungsausmaßes berücksichtigt bzw. eingehalten werden. So kann insbesondere der Roboter die Betätigungsfläche derart führen bzw. die Steuerung ihn derart regeln, dass ein oder mehrere Gelenke und/oder Körperglieder des Nutzers bei der robotergeführten Bewegung der Betätigungsfläche nur die vorgegebenen Bewegungsausmaße aufweisen.

Weitere Vorteile und Merkmale ergeben sich aus den Unteransprüchen und den Ausführungsbeispielen. Hierzu zeigt, teilweise schematisiert, die einzige:

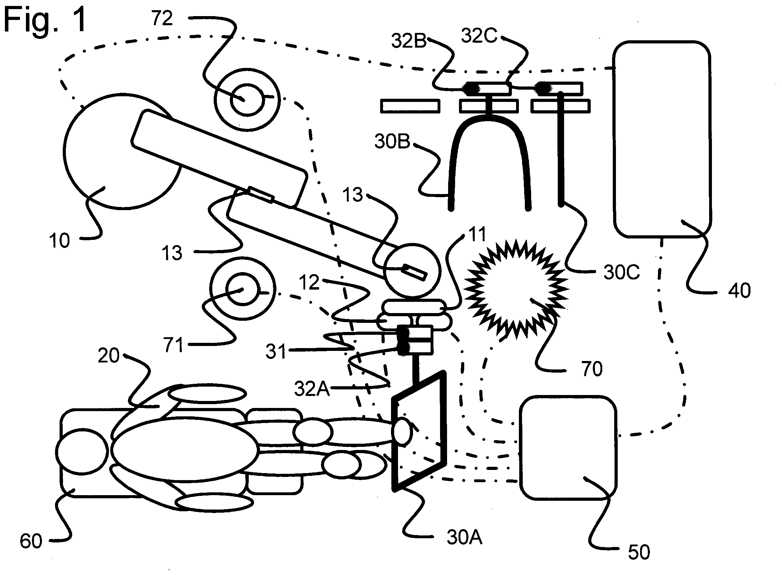

Fig. 1: ein Trainingssystem nach einer Ausführung der vorliegenden Erfindung.

Fig. 1 zeigt ein Trainingssystem nach einer Ausführung der vorliegenden Erfindung. Das Trainingssystem weist einen Roboter 10 auf. Der Roboter weist einen Arm mit sechs elektromotorisch aktuierten Drehgelenken mit paarweise aufeinander senkrecht stehenden oder parallelen Drehachsen auf.

Das Trainingssystem weist weiter mehrere unterschiedliche Betätigungsflächen 30A, 30B und 30C auf, die wahlweise lösbar an einem Roboterflansch 11 befestigt und somit durch den Roboter geführt werden. Der Roboterflansch 11 weist gegenüber einer umgebungsfesten Roboterbasis die durch die sechs Gelenke des Roboters definierten Freiheitsgrade auf.

Im Ausführungsbeispiel weist die aktuell angekoppelte bzw. robotergeführte Betätigungsfläche 30A eine Plattform zum Abstützen eines oder beider Füße eines Nutzers auf, so dass das Trainingssystem insbesondere, wie in Fig. 1 angedeutet, als sogenannte Funktionsstemme fungieren kann. Die Betätigungsflächen 30B, 30C sind hingegen als Griff zum Halten mit einer (30C) oder beiden Händen (30B) ausgebildet.

Das Trainingssystem weist ein Krafterfassungsmittel zum Ermitteln einer Kraft- und Momentenbeaufschlagung der Betätigungsfläche in jeweils drei zueinander orthogonalen Richtungen in Form eines sechsdimensionalen Kraft-/Momentensensors 12 auf, der zwischen dem Roboterflansch. 11 und der Betätigungsfläche 30A angeordnet ist.

Das Trainingssystem weist ein Aktivitätserfassungsmittel zum Ermitteln einer biomechanischen Belastung eines Nutzers 20 auf Basis der ermittelten Beaufschlagung der Betätigungsfläche sowie ein Steuermittel zum Regeln der Antriebe des Roboters 10 auf Basis einer vorgegebenen und der ermittelten biomechanischen Belastung des Nutzers auf, die beide in einer Steuerung 40 implementiert sind.

Im Ausführungsbeispiel, in dem der Roboter 10 als Funktionsstemme fungiert, werden beispielsweise in dem aktiven Kniegelenk des Nutzers 20 wirkende Kräfte und Momente auf Basis der ermittelten Beaufschlagung der Betätigungsfläche 30A auf Basis eines biomechanischen Modells ermittelt und mit vorgegebenen Belastungen verglichen. Dann regelt die Steuerung 40 den Roboter 10 derart, dass die im Kniegelenk des Nutzers 20 wirkenden Kräfte und Momente sich den gewünschten Belastungen näheren oder die zulässigen Belastungen nicht übersteigen.

Zusätzlich ermittelt die Steuerung auf Basis der ermittelten Beaufschlagung der Betätigungsfläche 30A auf Basis eines biomechanischen Modells eine muskuläre Beanspruchung im Kniestrecker, vergleicht diese mit einem vorgegebenen optimalen Trainingsreiz und regeln den Roboter 10 derart, dass die im Kniestrecker des Nutzers 20 wirkenden Kräfte sich der gewünschten Trainingsbelastung näheren.

Auf diese Weise kann vorteilhaft eine Überlastung des Kniegelenks vermieden und zugleich der Kniestrecker optimal ausbelastet werden.

Die Steuerung 40 weist mehrere biomechanische Modellmodule auf, die unterschiedliche Teile des Bewegungsapparates des Nutzers abbilden und unterschiedliche Komplexitätsgrade aufweisen. Die Steuerung 40 erstellt wahlweise, insbesondere je nach Trainingsplan, aus diesen Modulen jeweils das biomechanische Modell, auf dessen Basis sie dann die Belastung des Nutzers 20 ermittelt und den Roboter 10 regelt.

Die biomechanischen Modelle sind parametrierbar, um sie individuell an verschiedene Nutzer anzupassen. Die Parameter des Modells werden vom Nutzer oder einem Trainer eingegeben oder aus einer Datenbank ermittelt, insbesondere durch Identifikation einer Nutzeridentität und Abrufen von Parametern, die mit dieser Nutzeridentität verknüpft sind, aus einem Speichermittel der Steuerung 40. Zusätzlich oder alternativ können ein oder mehrere der Parameter auch durch das Trainingssystem selber ermittelt, insbesondere identifiziert oder abgeschätzt, werden.

Die Steuerung 40 berücksichtigt bei der Ermittlung der Belastung des Nutzers 20 zusätzlich eine Position von Referenzen des Nutzers, die im Ausführungsbeispiel durch umgebungsfeste Raumüberwachungssensoren, etwa eine Kamera 70 und entsprechende Bilderkennung, ermittelt werden. In einer nicht dargestellten Abwandlung kann die Position von Referenzen des Nutzers zusätzlich oder alternativ auch durch am Nutzer angeordnete Positionssensoren ermittelt werden. In einer ebenfalls nicht dargestellten Abwandlung kann die Steuerung 40 zusätzlich oder alternativ bei der Ermittlung der Belastung des Nutzers 20 auch eine Nerven-und/oder Muskelaktivität des Nutzers 20 berücksichtigt, die durch am Nutzer angeordnete EMG-Sensoren ermittelt wird.

Die Referenzen können eine bekannte Lage relativ zu Gelenken des Bewegungsapparates des Nutzers aufweisen, beispielsweise dem Kniegelenk. Dann kann die Steuerung 40 auf Basis der erfassten Position der Referenzen die Position des Kniegelenks ermitteln und auf deren Basis unter Berücksichtigung der Beaufschlagung der Betätigungsfläche 30A eine Belastung im Kniegelenk ermitteln.

Im Ausführungsbeispiel regelt die Steuerung 40 eine Kraft, die der Roboter 10 auf die robotergeführte Betätigungsfläche 30A ausübt, sowie eine Bewegung der robotergeführten Betätigungsfläche durch den Roboter auf Basis der vorgegebenen und der ermittelten biomechanischen Belastung des Nutzers.

Wird beispielsweise auf Basis der ermittelten Beaufschlagung der Betätigungsfläche 30A eine biomechanische Überlastung des Kniegelenkes des Nutzers 20 ermittelt, kann die Steuerung 40 die Kraft, mit der der Roboter 10 die Betätigungsfläche 30A beaufschlagt, insbesondere einer Bewegung durch den Nutzer entgegensetzt (konzentrisches Training), reduzieren und/oder ihre Richtung und/oder die Bewegungstrajektorie der Betätigungsfläche 30A so ändern, dass die biomechanische Belastung des Kniegelenkes reduziert wird, beispielsweise eine Bewegung der Betätigungsfläche 30A mit einer Bewegungsachse des Kniegelenks (besser) korreliert.

Das Trainingssystem weist ein Sicherheitsmittel mit einer Sicherheitssteuerung 50 zum Überwachen der Beaufschlagung der Betätigungsfläche 30A, der ermittelten biomechanischen Belastung des Nutzers und des Zustands des Roboters 10 auf.

Die Sicherheitssteuerung 50 erfasst zweikanalig die Beaufschlagung der Betätigungsfläche 30A mittels des Kraft-/Momentensensors 12 und den Zustand, insbesondere eine Position, Geschwindigkeit und/oder Beschleunigung, des Roboters 10 mittels Lichtschranken 71, 72. Zusätzlich vergleicht sie die ermittelte biomechanische Belastung des Nutzers 20 mit einer vorgegebenen zulässigen biomechanischen Belastung, beispielsweise maximal zulässigen Kräften im Knie. Falls die Sicherheitssteuerung 50 eine unzulässige Beaufschlagung der Betätigungsfläche 30A oder einen unzulässigen Zustand des Roboters 10 erfasst, beispielsweise eine auf die Betätigungsfläche 30A ausgeübte Kraft einen vorgegebenen Grenzwert übersteigt oder der Roboter 10 den durch die Lichtschranken 71, 72 vorgegebenen Arbeitsraum verlässt, oder falls die Sicherheitssteuerung 50 eine unzulässige biomechanische Belastung des Nutzers 20 erfasst, führt sie eine Ausgleichsbewegung der robotergeführten Betätigungsfläche 30A in eine vorgegebene Ausgangsstellung durch.

Zusätzlich oder alternativ zu den Lichtschranken 71, 72 und/oder der Kamera 70 kann die Sicherheitssteuerung 50 die Position des Roboters 10 auch durch Positions- bzw. Gelenkwinkelsensoren 13 an den Gelenken des Roboters erfassen.

Wie vorstehend bereits erwähnt, weist das Trainingssystem im Ausführungsbeispiel drei unterschiedliche Betätigungsflächen 30A - 30C auf, die wahlweise mit dem Roboter 10 koppelbar sind.

Die Steuerung 40 identifiziert die jeweils robotergeführte bzw. an den Roboterflansch 11 gekoppelte Betätigungsfläche (im Ausführungsbeispiel 30A) und regelt den Roboter 10 auf Basis der identifizierten robotergeführten Betätigungsfläche. Hierzu weisen die unterschiedlichen Betätigungsflächen 30A - 30C jeweils einen RFID-Transponder 32A, 32B bzw. 32C auf, der Roboter 10 ein Mittel 31 zum elektromagnetischen Erfassen des jeweils angekoppelten RFID-Transponders.

Das Trainingssystem weist eine Nutzer-Positioneinrichtung 60 mit einer verstellbaren Sitzfläche und Lehne auf.

Obwohl in der vorhergehenden Beschreibung exemplarische Ausführungen erläutert wurden, sei darauf hingewiesen, dass eine Vielzahl von Abwandlungen möglich ist. Außerdem sei darauf hingewiesen, dass es sich bei den exemplarischen Ausführungen lediglich um Beispiele handelt, die den Schutzbereich, die Anwendungen und den Aufbau in keiner Weise einschränken sollen. Vielmehr wird dem Fachmann durch die vorausgehende Beschreibung ein Leitfaden für die Umsetzung von mindestens einer exemplarischen Ausführung gegeben, wobei diverse Änderungen, insbesondere in Hinblick auf die Funktion und Anordnung der beschriebenen Bestandteile, vorgenommen werden können, ohne den Schutzbereich zu verlassen, wie er sich aus den Ansprüchen und diesen äquivalenten Merkmalskombinationen ergibt.

### Bezugszeichenliste

- 10: Roboter
- 11: Roboterflansch
- 12: Kraft-/Momentensensor
- 13: Gelenkwinkelsensor
- 20: Nutzer
- 30A; 30B, 30C: Betätigungsfläche
- 31: Mittel zum Erfassen eines RFID-Transponders
- 32A; 32B, 32C: RFID-Transponder
- 40: (Roboter)Steuerung (Aktivitätserfassungs- und Steuermittel)
- 50: Sicherheitssteuerung
- 60: Nutzer-Positioneinrichtung
- 70: Kamera (Raumüberwachungssensor)
- 71, 72: Lichtschranke

## Patentansprüche

1. Trainingssystem mit:
einem Roboter (10);
einer robotergeführten Betätigungsfläche (30A);
einem Aktivitätserfassungsmittel zum Ermitteln einer biomechanischen und/oder kardiovaskulären Belastung eines Nutzers (20), insbesondere auf Basis einer von einem Krafterfassungsmittel (12) des Trainingssystems ermittelten Beaufschlagung der Betätigungsfläche; und
einem Steuermittel zum Regeln des Roboters auf Basis einer vorgegebenen und der ermittelten biomechanischen und/oder kardiovaskulären Belastung des Nutzers; **dadurch gekennzeichnet, dass** das Trainingssystem ein Sicherheitsmittel (50) zum, insbesondere redundanten, Überwachen der Beaufschlagung der Betätigungsfläche, der ermittelten biomechanischen und/oder kardiovaskulären Belastung des Nutzers und/oder des Zustands des Roboters aufweist.

2. Trainingssystem nach Anspruch 1, wobei das Aktivitätserfassungsmittel dazu eingerichtet ist, die Belastung des Nutzers auf Basis wenigstens eines, insbesondere modularen und/oder parametrierbaren, biomechanischen und/oder kardiovaskulären Modells, und/oder eines ermittelten Zustands des Nutzers zu ermitteln.

3. Trainingssystem nach dem vorhergehenden Anspruch, wobei das Aktivitätserfassungsmittel dazu eingerichtet ist, den Zustand des Nutzers auf Basis einer erfassten Position, Beschleunigung, Nerven- und/oder Muskel- und/oder kardiovaskulären Aktivität und/oder Abmessung einer biologischen Struktur des Nutzers zu ermitteln.

4. Trainingssystem nach dem vorhergehenden Anspruch, wobei das Aktivitätserfassungsmittel wenigstens einen am Nutzer angeordneten, insbesondere inertialen, Positionssensor, Beschleunigungssensor, EMG-Sensor und/oder wenigstens einen Sensor zum Ermitteln eines kardiovaskulären Parameters und/oder wenigstens einen, insbesondere nicht-invasiven Sensor, zum Ermitteln einer Abmessung einer biologischen Struktur des Nutzers und/oder wenigstens einen Raumüberwachungssensor (70) aufweist.

5. Trainingssystem nach einem der vorhergehenden Ansprüche, wobei das Steuermittel dazu eingerichtet ist, eine Kraft, insbesondere Kraftrichtung und/oder -größe, des Roboters auf die robotergeführte Betätigungsfläche und/oder eine Bewegung der robotergeführten Betätigungsfläche durch den Roboter, insbesondere eine Bewegungsrichtung und/oder Geschwindigkeit, auf Basis der vorgegebenen und der ermittelten biomechanischen und/oder kardiovaskulären Belastung des Nutzers zu regeln.

6. Trainingssystem nach einem der vorhergehenden Ansprüche, wobei das Sicherheitsmittel dazu eingerichtet ist, eine Ausgleichsbewegung durchzuführen, falls eine unzulässige Beaufschlagung der Betätigungsfläche oder biomechanischen und/oder kardiovaskulären Belastung des Nutzers oder ein unzulässiger Zustand des Roboters ermittelt wird.

7. Trainingssystem nach einem der vorhergehenden Ansprüche, wobei das Steuermittel dazu eingerichtet ist, einen Nutzer (20), insbesondere berührungslos, zu identifizieren und den Roboters auf Basis des identifizierten Nutzers zu regeln.

8. Trainingssystem nach einem der vorhergehenden Ansprüche, mit wenigstens zwei Betätigungsflächen (30A, 30B, 30C), die wahlweise mit dem Roboter koppelbar sind, wobei das Steuermittel dazu eingerichtet ist, die robotergeführten Betätigungsflächen wenigstens teilweise automatisiert zu wechseln und/oder zu identifizieren und den Roboter auf Basis der identifizierten robotergeführten Betätigungsfläche zu regeln.

9. Trainingssystem nach einem der vorhergehenden Ansprüche, mit einem Fixiermittel zum Fixieren des Nutzers an der robotergeführten Betätigungsfläche und/oder einer Nutzer-Positioneinrichtung (60).

10. Trainingssystem nach einem der vorhergehenden Ansprüche, mit einem Ausgabemittel zum Ausgeben einer Rückmeldung auf Basis der ermittelten biomechanischen und/oder kardiovaskulären Belastung.

11. Verfahren zum Regeln des Roboters eines Trainingssystems nach einem der vorhergehenden Ansprüche, wobei eine biomechanische und/oder kardiovaskulären Belastung des Nutzers, insbesondere auf Basis einer ermittelten Beaufschlagung der Betätigungsfläche, ermittelt und der Roboter auf Basis einer vorgegebenen und der ermittelten biomechanischen und/oder kardiovaskulären Belastung des Nutzers geregelt wird.

12. Computerprogrammprodukt mit einem Programmcode, der dafür sorgt, dass eine Steuerung (40) eines Trainingssystems nach einem der Ansprüche 1-10 die Verfahrensschritte des Anspruchs 11 ausführt.

## Claims

1. A training system comprising:
a robot (10);
a robot-guided actuation surface (30A);
an activity detection means for determining a biomechanical and / or cardiovascular load of a user (20), in particular on the basis of a loading of the actuation surface determined by a force detection means (12) of the training system; and
a control means for controlling, in a closed-loop control manner, the robot on the basis of a biomechanical and / or cardiovascular load of the user specified in advance and the detected biomechanical and / or cardiovascular load of the user; **characterised in that** the training system comprises
a safety means (50) for monitoring the loading of the actuation surface, in particular for redundantly monitoring the loading of the actuation surface, the determined biomechanical and / or cardiovascular load of the user and / or the condition of the robot.

2. The training system according to claim 1, wherein the activity detection means is set up to determine the load of the user on the basis of at least one biomechanical and / or cardiovascular model, in particular on the basis of at least one modular and / or parameterisable biomechanical and / or cardiovascular model, and / or on the basis of at least one determined condition of the user.

3. The training system according to the preceding claim, wherein the activity detection means is set up to determine the condition of the user on the basis of a detected position, a detected acceleration, a detected nerve activity and / or a detected muscle activity and / or a detected cardiovascular activity and / or a dimension of a biological structure of the user.

4. The training system according to the preceding claim, wherein the activity detection means comprises at least one position sensor, acceleration sensor or EMG sensor arranged on the user, in particular at least one inertial position sensor, acceleration sensor or EMG sensor arranged on the user and / or at least one sensor for determining a cardiovascular parameter and / or at least one sensor for determining a dimension of a biological structure of the user, in particular at least one non-invasive sensor for determining a dimension of a biological structure of the user and / or at least one room monitoring sensor (70).

5. The training system according to any one of the preceding claims, wherein the control means is set up to control, in a closed-loop control manner, a force of the robot on the robot-guided actuation surface, in particular a direction and / or a magnitude of a force of the robot on the robot-guided actuation surface and / or a movement, by the robot, of the robot-guided actuation surface, in particular a direction of movement and / or a velocity of the robot-guided actuation surface, on the basis of the biomechanical and / or cardiovascular load of the user specified in advance and the determined biomechanical and / or cardiovascular load of the user.

6. The training system according to any one of the preceding claims, wherein the safety means is set up to carry out a compensatory movement if a loading of an unacceptable amount on the actuation surface or an unacceptable biomechanical and / or cardiovascular load of the user or an unacceptable condition of the robot is detected.

7. The training system according to any one of the preceding claims, wherein the control means is set up to identify a user (20), in particular in a contactless manner, and to control, in a closed-loop control manner, the robot on the basis of the identified user.

8. The training system according to any one of the preceding claims, comprising at least two actuation surfaces (30A, 30B, 30C) which can selectively be coupled to the robot, wherein the control means is set up to change and / or identify the robot-guided actuation surfaces at least in a partially automated manner and to control, in a closed-loop control manner, the robot on the basis of the identified robot-guided actuation surface.

9. The training system according to any one of the preceding claims, comprising a fixing means for fixing the user to the robot-guided actuation surface and / or to a user positioning device (60).

10. The training system according to any one of the preceding claims, comprising an output means for outputting feedback on the basis of the determined biomechanical and / or cardiovascular load.

11. A method of controlling, in a closed-loop control manner, the robot of a training system according to any one of the preceding claims, wherein a biomechanical and / or a cardiovascular load of the user is determined, in particular on the basis of a determined loading of the actuation surface, and the robot is controlled, in a closed-loop control manner, on the basis of a biomechanical and / or cardiovascular load of the user specified in advance and the detected biomechanical and / or cardiovascular load of the user.

12. A computer program product comprising a program code which is arranged to cause a control facility (40) of a training system according to any one of the claims 1 - 10 to carry out the method steps of the claim 11.

## Revendications

1. Système d'entraînement avec :
un robot (10) ;
une surface d'actionnement (30A) guidée par robot ;
un moyen de détection d'activité pour la détermination d'une sollicitation biomécanique et/ou cardiovasculaire d'un utilisateur (20), en particulier sur la base d'une alimentation déterminée par un moyen de détection de force (12) du système d'entraînement de la surface d'actionnement ; et
un moyen de commande pour la régulation du robot sur la base d'une sollicitation prédéfinie et de la sollicitation biomécanique et/ou cardiovasculaire déterminée de l'utilisateur ; **caractérisé en ce que** le système d'entraînement présente un moyen de sécurité (50) pour la surveillance, en particulier redondante de l'alimentation de la surface d'actionnement, de la sollicitation biomécanique et/ou cardiovasculaire déterminée de l'utilisateur et/ou de l'état du robot.

2. Système d'entraînement selon la revendication 1, dans lequel le moyen de détection d'activité est conçu afin de déterminer la sollicitation de l'utilisateur sur la base d'au moins un modèle en particulier modulaire et/ou paramétrable, biomécanique et/ou cardiovasculaire, et/ou d'un état déterminé de l'utilisateur.

3. Système d'entraînement selon la revendication précédente, dans lequel le moyen de détection d'activité est conçu afin de déterminer l'état de l'utilisateur sur la base d'une position, accélération, activité nerveuse et/ou musculaire et/ou cardiovasculaire et/ou dimension détectée d'une structure biologique de l'utilisateur.

4. Système d'entraînement selon la revendication précédente, dans lequel le moyen de détection d'activité présente au moins un capteur de position, capteur d'accélération, capteur EMG en particulier inertiel, agencé sur l'utilisateur et/ou au moins un capteur pour la détermination d'un paramètre cardiovasculaire et/ou au moins un capteur en particulier non invasif pour la détermination d'une dimension d'une structure biologique de l'utilisateur et/ou au moins un capteur de surveillance spatial (70).

5. Système d'entraînement selon l'une quelconque des revendications précédentes, dans lequel le moyen de commande est conçu afin de réguler une force, en particulier un sens et/ou une grandeur de force du robot sur la surface d'actionnement guidée par robot et/ou un mouvement de la surface d'actionnement guidée par robot par le robot, en particulier un sens de mouvement et/ou une vitesse, sur la base de la sollicitation prédéfinie et la sollicitation biomécanique et/ou cardiovasculaire déterminée de l'utilisateur.

6. Système d'entraînement selon l'une quelconque des revendications précédentes, dans lequel le moyen de sécurité est conçu afin de réaliser un mouvement de compensation en cas où une alimentation non autorisée de la surface d'actionnement ou sollicitation biomécanique et/ou cardiovasculaire de l'utilisateur ou un état non autorisé du robot est déterminé.

7. Système d'entraînement selon l'une quelconque des revendications précédentes, dans lequel le moyen de commande est conçu afin d'identifier un utilisateur (20), en particulier sans contact et de réguler le robot sur la base de l'utilisateur identifié.

8. Système d'entraînement selon l'une quelconque des revendications précédentes, avec au moins deux surfaces d'actionnement (30A, 30B, 30C) qui peuvent être couplées au choix avec le robot, dans lequel le moyen de commande est conçu afin de changer et/ou d'identifier de manière au moins partiellement automatisée les surfaces d'actionnement guidées par robot et de réguler le robot sur la base de la surface d'actionnement guidée par robot identifiée.

9. Système d'entraînement selon l'une quelconque des revendications précédentes, avec un moyen de fixation pour la fixation de l'utilisateur à la surface d'actionnement guidée par robot et/ou d'un dispositif de positionnement d'utilisateur (60).

10. Système d'entraînement selon l'une quelconque des revendications précédentes, avec un moyen de sortie pour la sortie d'un retour sur la base de la sollicitation biomécanique et/ou cardiovasculaire déterminée.

11. Procédé de régulation du robot d'un système d'entraînement selon l'une quelconque des revendications précédentes, dans lequel une sollicitation biomécanique et/ou cardiovasculaire de l'utilisateur est déterminée en particulier sur la base d'une alimentation déterminée de la surface d'actionnement et le robot est régulé sur la base d'une sollicitation prédéfinie et de la sollicitation biomécanique et/ou cardiovasculaire déterminée de l'utilisateur.

12. Produit de programme informatique avec un code de programme qui veille à ce qu'une commande (40) d'un système d'entraînement selon l'une quelconque des revendications 1 à 10 réalise les étapes de procédé de la revendication 11.
